# EUROPEAN PATENT SPECIFICATION

(11) **EP 0 973 717 B1**
(45) Date of publication and mention of the grant of the patent: **01.10.2003**
(21) Application number: 98914509.9
(22) Date of filing: 07.04.1998
(51) Int. Cl.: C07C 51/46, C07C 53/08

(54) **WATER SEPARATION PROCESS**
VERFAHREN ZUR WASSERABTRENNUNG
PROCEDE DE SEPARATION DE L'EAU

(30) Priority: 09.04.1997 GB 9707177; 11.04.1997 US 43841 P
(43) Date of publication of application: 26.01.2000
(73) Proprietor: E.I. DU PONT DE NEMOURS AND COMPANY, Wilmington Delaware 19898 (US)
(72) Inventor: PARTEN, William, David, Stockton on Tees TS17 0PD (GB)
(74) Representative: Carpmaels & Ransford
(86) International application number: US9806740
(87) International publication number: WO98045239

(56) References cited:
- WO-A-96/06065
- WO-A-97/29068
- GB-A- 273 744
- US-A- 4 250 330

## Description

This invention relates to the separation of water from a liquid phase medium containing water and at least one organic component, e.g. the separation of water from an aqueous feed stream containing an aliphatic carboxylic acid, such as acetic acid.

In the production of terephthalic acid, for example, by the liquid phase oxidation of p-xylene in a solvent such as acetic acid, water and solvent are typically removed as an overhead vapor stream from the oxidation reactor as one means for controlling the heat of reaction. The vapor stream so removed can contain other components, such as, for example, gases (e.g. unreacted oxygen, carbon oxides and nitrogen) and organic components, such as xylene and methyl acetate, some of which are formed as a by-product of the reaction resulting from oxidation of the acetic acid solvent. The vapor stream is condensed to recover condensibles, some of which may be recycled as reflux to the oxidation reactor while other of the condensibles are passed to a separation process which, in turn, allows for recovery of acetic acid having a reduced water content. The feed stream to the separation process tends to contain methyl acetate along with acetic acid and water.

There are a number of options available for the handling of the methyl acetate that is recovered from the separation process. It may be recycled back to the reactor where it is believed to suppress oxidation of the acetic acid solvent. It may be hydrolyzed to methanol and acetic acid which may be recycled to the oxidation reactor, or the methyl acetate may be recovered to use as a fuel or for some other chemical utility.

U.S. Patent No. 4,250,330 discloses a process for producing terephthalic acid in which the oxidation reactor overhead vapor stream is condensed and the liquid condensate which results, consisting of acetic acid and water, is fed to an azeotropic distillation system in which the azeotropic agent is iso-butyl acetate. The vapor phase leaving the azeotropic distillation column comprising water, entrainer and methyl acetate is totally condensed to form an aqueous phase and an organic phase without forming a vapor phase. Iso-butyl acetate and methyl acetate are subsequently recovered from the aqueous phase in a stripping zone.

WO 96/06065 discloses a process for producing terephthalic acid in which the condensate from condensing the reactor overheads vapor stream contains a relatively high concentration of water, i.e., in the range of from 20% to 40% by wt. water. Azeotropic distillation is used to separate the water from acetic acid using as entrainer iso-butyl acetate, n-propyl acetate, or an entrainer with a boiling point intermediate those of iso-butyl acetate and n-propyl acetate. The distillation is operated using a single organic phase reflux.

Although one convenient form of separation process comprises azeotropic distillation, which is preferred over fractional distillation because of its improved energy efficient in operation, the presence of methyl acetate in the distillation column tends to interfere with distillation by reducing the amount of the aqueous phase formed from the condensation of the overheads from the column, which, in turn, increases energy consumption and the diameter of the column. With a high enough concentration of methyl acetate in the overheads vapor stream from the distillation column, the vapor phase will tend to condense to a single liquid phase making recovery of water from the overhead product impractical.

The present invention provides a method for managing methyl acetate, produced as a by-product in the course of producing an aromatic carboxylic acid by liquid phase oxidation of the corresponding precursor in acetic acid, especially in circumstances where at least partial recycle of the impurity to the reaction is desirable and where solvent and water from the reaction are to be separated using azeotropic distillation.

### SUMMARY OF THE INVENTION

The present invention provides a process for azeotropic distillation of a feedstock in which the feedstock comprises acetic acid, water and an ester impurity which is methyl acetate, which comprises:
(a) conducting the azeotropic distillation in the presence of an organic entrainer selected from n-propyl acetate and iso-butyl acetate to produce a liquid phase component comprising said acetic acid having a reduced water content relative to the water content in the feedstock and a vapor phase component which contains the organic entrainer, water and methyl acetate;
(b) condensing the vapor phase component;
(c) separating the condensate into (1) an organic phase comprising primarily entrainer with minor amounts of water and methyl acetate and (2) an aqueous phase comprising primarily water with minor amounts of entrainer and methyl acetate; and
(d) returning the organic phase to the distillation column, characterized by controlling condensation of the vapor phase component upstream of condensate phase separation whereby methyl acetate and a minor amount of entrainer remain in the vapor phase.

The azeotropic distillation process is carried out according to the invention using an organic entrainer which is an ester selected from n-propyl acetate and iso-butyl acetate.

In one embodiment of the invention, the vapor phase component, which comprises the organic entrainer, water and the methyl acetate is removed from the distillation process as a tops product, i.e., an overhead vapor stream, from the distillation column.

In an alternative embodiment of the invention, the azeotropic distillation column is extended, i.e., it can be elongated, so that the vapor phase component comprising the organic entrainer, water and the methyl acetate pass into the extended section and undergo further distillation whereby the organic entrainer and water are condensed and recovered from the column as a liquid phase, and the methyl acetate remains in the vapor phase. The recovered organic entrainer/water liquid phase can then be allowed to phase separate into said organic and aqueous phases.

Thus the invention also provides a process for azeotropic distillation of a feedstock in which the feedstock comprises acetic acid, water and an ester impurity which is methyl acetate, which comprises:
(a) conducting the azeotropic distillation in the presence of an organic entrainer selected from n-propyl acetate and iso-butyl acetate in a distillation zone to produce a liquid phase component comprising said acetic acid having a reduced water content relative to the water content in the feedstock and a vapor phase component which contains the organic entrainer, water and methyl acetate;
(b) characterized by passing the vapor phase component to an incorporated separation zone, and therein separating and recovering a substantial fraction of methyl acetate as a vapor while simultaneously collecting water, entrainer and a minor amount of methyl acetate as a liquid phase;
(c) separating the resulting liquid phase into (1) an organic phase comprising primarily entrainer with minor amounts of water and methyl acetate and (2) an aqueous phase comprising primarily water with a minor amount of methyl acetate; and
(d) returning the organic phase to the distillation zone.

According to another aspect, the present invention provides a process for producing an aromatic carboxylic acid, preferably terephthalic acid, by liquid phase oxidation of a precursor of said acid, i.e., paraxylene, in acetic acid, in the presence of a catalyst system, which comprises:
(a) removing from the oxidation reaction an overheads vapor stream which comprises the acetic acid, water produced in the oxidation reaction and methyl acetate formed as a by-product of the oxidation reaction;
(b) deriving from the overheads vapor stream a first liquid phase component which contains the acetic acid, water and methyl acetate;
(c) azeotropically distilling said first liquid phase component in the presence of an organic entrainer selected from n-propyl acetate and isobutyl acetate to produce a second liquid phase component comprising said acetic acid having a reduced water content relative to the water content in the first liquid phase and a vapor phase component which contains the organic entrainer, water and methyl acetate;
(d) condensing the vapor phase component under conditions whereby the organic entrainer and water condense while a substantial portion of the methyl acetate remains in the vapor phase;
(e) recovering said methyl acetate in the vapor phase;
f) separating the organic entrainer and water condensate from step (d) into an organic phase and an aqueous phase;
(g) returning said organic phase to the distillation column; and
(h) returning at least part of said recover methyl acetate to the liquid phase oxidation reaction.

The improved process according to the invention makes it economically feasible on a commercial scale to recycle to the oxidation reactor substantially all of the methyl acetate that formed as a by-product during the oxidation reaction and is withdrawn from the reaction zone via an overheads vapor stream and thereby establish a standing concentration of the methyl acetate such that the production of the impurity in the oxidation reactor, i.e., the oxidation reaction zone, is balanced by its destruction. Thus, a substantial level of methyl acetate can be recycled to the oxidation reactor without suffering a reduction in energy savings otherwise made possible by using azeotropic distillation to separate water from the acetic acid.

### BRIEF DESCRIPTION OF THE DRAWINGS

Fig. 1 is a schematic process diagram which illustrates one embodiment of the invention in which water is separated from acetic acid solvent via azeotropic distillation, and methyl acetate is recycled.
Fig. 2 is a schematic diagram of an alternative embodiment of the invention.

### DETAILED DESCRIPTION

The present invention is an improved process for producing an aromatic carboxylic acid, particularly terephthalic acid, by liquid phase oxidation of a precursor of the acid, i.e., paraxylene, in acetic acid, in the presence of a catalyst system, wherein the process comprises:
(a) controlling the heat of reaction by removing from the oxidation reaction zone an overheads vapor stream which comprises the acetic acid, water produced in the reaction and methyl acetate formed as a by-product of the oxidation reaction which is an ester derivative of the aliphatic acid solvent;
(b) deriving from the overheads vapor stream a first liquid phase component which contains the acetic acid, water and methyl acetate;
(c) azeotropically distilling said first liquid phase in the presence of an organic entrainer selected from n-propyl acetate and iso-butyl acetate to produce a second liquid phase component comprising said acetic acid having a reduced water content relative to the water content in the first liquid phase and a vapor phase component which contains the organic entrainer, water and methyl acetate;
(d) condensing the vapor phase component under conditions whereby the organic entrainer and water condense while a substantial portion of the methyl acetate remains in the vapor phase;
(e) recovering the methyl acetate in the vapor phase;
(f) separating the organic entrainer and water condensate from step (d) into an organic phase and an aqueous phase;
(g) returning said organic phase to the distillation column; and
(h) returning at least part of said recovered methyl acetate to the oxidation reaction zone.

The first liquid phase component derived from the overheads vapor stream may be isolated by condensing a substantial proportion of the condensible components of the overheads stream in one or more stages. Depending on how the process is to be operated, the first liquid phase may comprise a relatively water rich, carboxylic acid lean fraction (water draw off stream) of the condensate, with a corresponding relatively water lean, carboxylic acid rich fraction. The carboxylic acid rich fraction is recycled as a reflux stream to the oxidation reaction, i.e. reaction zone or oxidation reactor. In the case where the carboxylic acid rich fraction is recycled as a reflux to the oxidation reaction, it will be understood that part of the methyl acetate will necessarily by-pass azeotropic distillation and be returned directly to the oxidation reaction via the reflux stream. Thus, where reference is made above to recycling all of the methyl acetate to the oxidation reaction, some may be recycled via azeotropic distillation and some via the reflux stream. In addition, in practice it has been observed that some of the methyl acetate may remain in vapor form following said condensation of the overheads stream. This fraction of the methyl acetate may be recovered in an absorber and, if desired, also recycled to the oxidation reaction.

The aromatic carboxylic acid production processes in which the instant invention is most applicable are those processes employed on a commercial scale for production of terephthalic acid and isophthalic acid.

In practice, the feedstock supplied to the azeotropic distillation column can also include aqueous feeds derived from other aspects of the process for the aromatic carboxylic acid, such as, for example, the purification step as described in EP-A-498501, the teachings of which are incorporated herein by reference. Thus, the aromatic carboxylic acid produced by the oxidation reaction referred to above may be dissolved in water to form an aqueous solution and then purified by hydrogenation under elevated temperature and pressure conditions. Following hydrogenation, the purified aromatic carboxylic acid is separated from the mother liquor, and the mother liquor is supplied to the azeotropic distillation column as such and/or after further treatment (involving, for instance, precipitation and separation of less pure aromatic carboxylic acid therefrom).

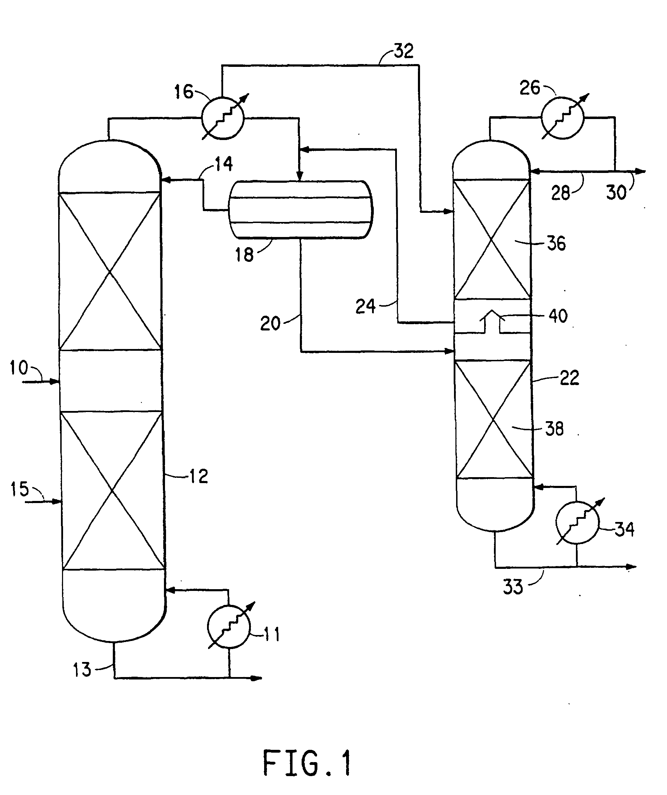

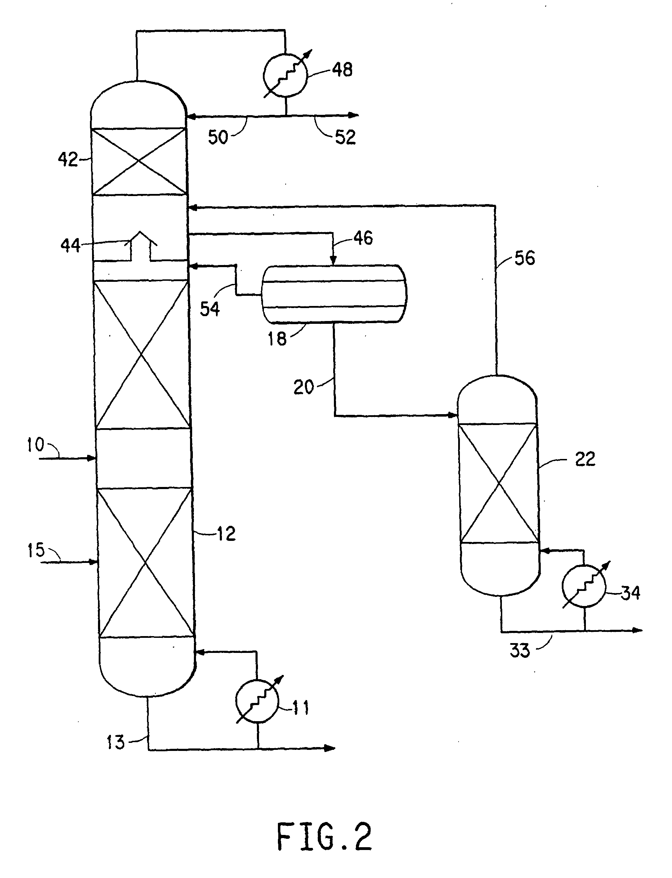

The invention will now be illustrated by the use of examples based on computer simulations with references to Figs. 1 and 2. Four cases have been examined in which water was separated from acetic acid using normal propyl acetate as the organic entrainer with varying amounts of methyl acetate present and varying column configurations.

The water draw off and combined feeds to a separations train are summarized in Table 1 and the results from the simulations are summarized in Table 2. Referring to Fig. 1, a feed stream 10 to distillation column 12 (which may be a packed or a tray type column) comprises the water draw off obtained from the oxidation reactor overheads condenser system associated with the oxidation reactor for the production of terephthalic acid. Some condensate remaining after water draw off is typically recycled directly to the reactor. In addition, a further feed stream 15 is shown which is intended to represent combined feeds to the distillation column 12 from other parts of the plant or production process. Heat to column 12 is supplied via reboiler 11. A low boiling point organic entrainer, such as n-propyl acetate, is supplied to the column via line 14, and the column is operated so as to insure penetration of the entrainer to a level below feed 10 whereby feed 10 enters the column at an entrainer-rich region. Feed 15 represents a combination of other possible aqueous feeds (liquid or vapor) that may be introduced to other points along the height of the column either within or below the azeotropic zone. These feeds may be derived from a high pressure absorber and/or a first and second crystallizer which may also be associated with the oxidation reactor or the feeds may come from a purification process. In operation, the primary feed will be the water draw off stream derived from the reactor overheads system which will generally contribute more water than any of the other feeds which may optionally be present. In some cases, such other feeds represented within stream 15 may be combined with feed from the reactor overheads condenser system and introduced into column 12 as a single feed.

The bottoms product withdrawn from the base of column 12 via line 13 comprises acetic acid having a relatively low water content relative to the incoming feed stream 10 and is suitable for recycle to the oxidation reaction. The tops product at the head of column 12 is cooled in a column overheads condenser system 16, and the condensate is supplied to phase separator 18 where condensate is separated into an organic phase (primarily organic entrainer with a small quantity of water and some methyl acetate, paraxylene and other organics) and an aqueous phase containing a small quantity of entrainer and, *inter alia,* some methyl acetate. The organic phase comprising mainly entrainer is reintroduced into the distillation column via line 14 while the aqueous phase is passed via line 20 to a stripping column 22 for recovery of entrainer via line 24 for recycle to the azeotropic distillation column and recovery of methyl acetate as a vapor phase tops product which is condensed in condenser 26. Part of the recovered methyl acetate is refluxed to the stripping column via line 28, and the remainder is recovered via line 30.

In stripping column 22, heat is supplied by reboiler 34, and the column comprises upper and lower packed or trayed sections 36 and 38, respectively, with the aqueous phase from the separator 18 being fed to the column via line 20. A separator device 40 is provided within stripping column 22 which allows upward passage of vapor in column 22 but which also prevents downward passage of the liquid phase. Separator device 40 serves to collect an entrainer rich fraction which is returned via line 24 to the phase separator. Alternatively, the entrainer rich fraction recovered from device 40 may be processed to remove water therefrom (not shown), and the entrainer can then be passed directly into column 12, e.g., via reflux line 14. The bottoms product obtained from the stripping stripping column comprises water, which is largely free of entrainer, and organics and is removed via line 33.

As thus far described, the scheme of Fig. 1 can cope with feed streams in which the methyl acetate content is low, typically where the methyl acetate content is from 30 to 100 times less than the water content on a weight basis. However, if it is desired to recycle methyl acetate to the oxidation reaction zone, the methyl acetate concentration of the water draw off stream derived from the reactor overheads condenser system (i.e., line 10) will tend to be relatively high (typically in the range from 2% to 10%, but usually 4% to 9%, on a weight basis of the draw off stream) and will be detrimental to efficient, i.e., optimum economical, operation of the azeotropic distillation column. In particular, the amount of aqueous phase from condensation of the overheads phase from column 12 will tend to decrease causing the heat load in the column to increase. Consequently there is a serious disincentive to use azeotropic distillation in circumstances where recycle of methyl acetate to the oxidation reactor is desired. This is illustrated by comparing Case 1 and Case 2 in Table 2 which were modeled with operation as described above.

The problem arising when high concentrations of methyl acetate are present in feed stream 10 is overcome in the full embodiment of Fig. 1 by controlling the condensation step upstream of phase separator 18 to allow for a methyl acetate rich vapor stream to be obtained whereby the amount of methyl acetate routed to phase separator 18 via the condensate can be reduced to manageable levels without materially affecting the efficiency of operation of the azeotropic distillation process. Thus, as shown in Fig. 1, condenser system 16 is operated under conditions whereby a vapor phase side stream 32, which is rich in methyl acetate, is obtained, side stream 32 being supplied directly to stripping column 22 as shown for recovery of the methyl acetate and entrainer contained in the side stream. The benefit of operating with a vapor side stream 32, from condenser 16 to a stripping column 22 is illustrated via Case 3 in Table 2.

Fig. 2 illustrates an alternative embodiment of the invention which is generally similar to Fig. 1 such that like reference numerals are used to identify components which function in the manner described with reference to the embodiment of Fig. 1. In the embodiment of Fig. 2, column 12 is extended, i.e., elongated, to incorporate section 42 in the upper portion thereof in which a substantial fraction of the methyl acetate is separated as a vapor phase and in which a separator device 44 is installed to collect entrainer, water and a small amount of methyl acetate as a liquid phase for supply via line 46 to phase separator 18. The overheads vapor stream from section 42 is passed to overheads condenser 48 to condense methyl acetate, and the condensate is, in part, refluxed via line 50 and ,in part, removed for recycle to the oxidation reaction via line 52 as shown.

Separator 18 serves to separate the liquid phase supplied via line 46 into an organic phase, which is reintroduced via line 54 into column 12 at a point below the separator device 44 and an aqueous phase which is passed via line 20 to stripping column 22. Compared to the embodiment of Fig. 1, the embodiment of Fig. 2 does not require an overheads condensing system associated with stripping column 22 for recovery of the methyl acetate. Instead, the vapor phase rich in methyl acetate is recycled via line 56 to column 12 at section 42 of this embodiment, and this has been modeled as Case 4 in Table 2.

**Table 1**

| Case Number and Streams | Acetic Acid Flow kg/hr | Water Flow kg/hr | Methyl Acetate Flow kg/hr |
|---|---|---|---|
| Water Draw Off - 1 | 57,115 | 20,762 | 360 |
| Water Draw Off - 2 | 57,115 | 20,762 | 1983 |
| Water Draw Off - 3 | 57,115 | 20,762 | 1983 |
| Water Draw Off - 4 | 57,115 | 20,762 | 1983 |
| Combined Feeds - 1 | 117,158 | 28,789 | 440 |
| Combined Feeds - 2 | 117,158 | 28,789 | 2357 |
| Combined Feeds - 3 | 117,158 | 28,789 | 2357 |
| Combined Feeds - 4 | 117,158 | 28,789 | 2357 |

**Table 2**

| | Case 1* | Case 2* | Case 3 | Case 4 |
|---|---|---|---|---|
| Combined Heat load (MW) | 22.15 | 34.2 | 23.01 | 21.83 |
| Methyl Acetate Recovered (kg/hr) | 421 | 2347 | 2335 | 2336 |
| Methyl Acetate Percent of Organic Phase from Decanter | 6.9 | 32.4 | 6.3 | 1.5 |
| Water Percent of Organic Phase from Decanter | 3.4 | 3.6 | 3.4 | 3.4 |
| Percent Aqueous Phase from Decanter | 13.34 | 10.92 | 14.09 | 13.12 |

| | | | | |
|---|---|---|---|---|
| * Comparative Run | | | | |

## Claims

1. A process for azeotropic distillation of a feedstock in which the feedstock comprises acetic acid, water and an ester impurity which is methyl acetate, which comprises:
(a) conducting the azeotropic distillation in the presence of an organic entrainer selected from n-propyl acetate and iso-butyl acetate to produce a liquid phase component comprising said acetic acid having a reduced water content relative to the water content in the feedstock and a vapor phase component which contains the organic entrainer, water and methyl acetate;
(b) condensing the vapor phase component;
(c) separating the condensate into (1) an organic phase comprising primarily entrainer with minor amounts of water and methyl acetate and (2) an aqueous phase comprising primarily water with minor amounts of entrainer and methyl acetate; and
(d) returning the organic phase to the distillation column, **characterized by** controlling condensation of the vapor phase component upstream of condensate phase separation whereby methyl acetate and a minor amount of entrainer remain in the vapor phase.

2. The process of Claim 1 further **characterized by** simultaneously stripping the aqueous phase and the vapor phase in a stripping zone to recover water as a bottoms product, a liquid fraction which is entrainer-rich, and a vapor fraction comprising methyl acetate.

3. The process of Claim 2 further **characterized by** condensing the vapor fraction and recycling at least part of said vapor fraction condensate as reflux to the stripping zone.

4. A process for azeotropic distillation of a feedstock in which the feedstock comprises acetic acid, water and an ester impurity which is methyl acetate, which comprises:
(a) conducting the azeotropic distillation in the presence of an organic entrainer selected from n-propyl acetate and iso-butyl acetate in a distillation zone to produce a liquid phase component comprising said acetic acid having a reduced water content relative to the water content in the feedstock and a vapor phase component which contains the organic entrainer, water and methyl acetate;
(b) **characterized by** passing the vapor phase component to an incorporated separation zone, and therein separating and recovering a substantial fraction of methyl acetate as a vapor while simultaneously collecting water, entrainer and a minor amount of methyl acetate as a liquid phase;
(c) separating the resulting liquid phase into (1) an organic phase comprising primarily entrainer with minor amounts of water and methyl acetate and (2) an aqueous phase comprising primarily water with a minor amount of methyl acetate; and
(d) returning the organic phase to the distillation zone.

5. The process of Claim 4 further **characterized by** stripping the aqueous phase in a stripping zone to recover water as a bottoms product and a vapor fraction comprising methyl acetate.

6. The process of Claim 5 further **characterized by** recycling the vapor fraction as reflux to the separation zone.

7. A process for producing an aromatic carboxylic acid by liquid phase oxidation of a precursor of said acid in acetic acid in the presence of a catalyst system, which comprises:
(a) removing from the oxidation reaction an overheads vapor stream which comprises the acetic acid, water produced in the oxidation reaction and methyl acetate formed as a by-product of the oxidation reaction;
(b) deriving from the overheads vagor stream a first liquid phase component which contains the acetic acid, water and methyl acetate;
(c) azeotropically distilling said first liquid phase component in the presence of an organic entrainer selected from n-propyl acetate and iso-butyl acetate to produce a second liquid phase component comprising said acetic acid having a reduced water content relative to the water content in the first liquid phase and a vapor phase component which contains the organic entrainer, water and methyl acetate;
(d) condensing the vapor phase component under conditions whereby the organic entrainer and water condense while a substantial portion of the methyl acetate remains in the vapor phase;
(e) recovering said methyl acetate in the vapor phase;
(f) separating the organic entrainer and water condensate from step (d) into an organic phase and an aqueous phase;
(g) returning said organic phase to the column; and
(h) returning at least part of said recovered methyl acetate to the liquid phase oxidation reaction.

8. The process of Claim 7 in which the aromatic carboxylic acid is terephthalic acid, the precursor of said acid is paraxylene.

## Patentansprüche

1. Verfahren für eine azeotrope Destillation eines Aufgabegutes, bei welchem das Aufgabegut Essigsäure, Wasser und eine Esterverunreinigung umfasst, welch letztere aus einem Methylacetat besteht; Verfahren welches umfasst:
(a) ein Durchführen einer azeotropen Destillation in Anwesenheit eines organischen Schleppmittels, das ausgewählt wird zwischen n-Propylacetat und Isobutylacetat, um eine flüssige Phasenkomponente zu erzeugen, welche die Essigsäure umfasst, welche einen verminderten Wassergehalt relativ zu dem Wassergehalt in dem Aufgabegut aufweist, sowie eine dampfförmige Phasenkomponente, welche das organische Schleppmittel, Wasser und Methylacetat enthält;
(b) ein Kondensieren der dampfförmigen Phasenkomponente;
(c) eine Trennung des Kondensats in (1) eine organische Phase, welche in erster Linie ein Schleppmittel mit geringeren Mengen an Wasser und Methylacetat enthält, und (2) eine wässrige Phase, welche in erster Linie Wasser mit geringeren Mengen an Schleppmittel und Methylacetat enthält; und
(d) ein Zurückführen der organischen Phase in die Destillationssäule, **gekennzeichnet durch** ein Steuern der Kondensation der dampfförmigen Phasenkomponente stromaufwärts zu der Trennung der Kondensatphasen, wobei Methylacetat und eine geringere Menge an Schleppmittel in der dampfförmigen Phase verbleiben.

2. Verfahren gemäß Anspruch 1, weiterhin **gekennzeichnet durch** ein gleichzeitiges Strippen der wässrigen Phase und der dampfförmigen Phase in einer Strippingzone zum Zurückgewinnen von Wasser als ein Bodenprodukt, einer flüssigen Fraktion, die reich an Schleppmittel ist und einer dampfförmigen Fraktion, die Methylacetat enthält.

3. Verfahren gemäß Anspruch 2, weiterhin **gekennzeichnet durch** eine Kondensation der dampfförmigen Fraktion und **durch** ein Zurückführen von mindestens einem Teil des Kondensats dieser dampfförmigen Fraktion als einen Rückfluss zu der Strippingzone.

4. Verfahren für eine azeotrope Destillation eines Aufgabegutes, bei welchem das Aufgabegut Essigsäure, Wasser und eine Esterverunreinigung umfasst, welch letztere aus einem Methylacetat besteht, Verfahren welches umfasst:
(a) ein Durchführen der azeotropen Destillation in Anwesenheit eines organischen Schleppmittels, das ausgewählt wird zwischen n-Propylacetat und Isobutylacetat in einer Destillationszone, um eine flüssige Phasenkomponente zu erzeugen, welche die Essigsäure umfasst, welche einen verminderten Wassergehalt relativ zu dem Wassergehalt in dem Aufgabegut aufweist, sowie eine dampfförmige Phasenkomponente, welche das organische Schleppmittel, Wasser und Methylacetat enthält;
(b) **gekennzeichnet durch** ein Überführen der dampfförmigen Phasenkomponente zu einer eingebundenen Trennzone und darin ein Trennen und ein Wiedergewinnen einer wesentlichen Fraktion des Methylacetats als ein Dampf, während gleichzeitig Wasser, Schleppmittel und eine kleinere Menge an Methylacetat als eine flüssige Phase gesammelt wird;
(c) eine Trennung der resultierenden flüssigen Phase in (1) eine organische Phase, welche in erster Linie Schleppmittel mit geringeren Mengen an Wasser und Methylacetat enthält, und (2) eine wässrige Phase, welche in erster Linie Wasser mit einer geringeren Menge an Methylacetat enthält; und
(d) ein Zurückführen der organischen Phase in die Destillationszone.

5. Verfahren gemäß Anspruch 4, weiterhin **gekennzeichnet durch** ein Strippen der wässrigen Phase in eine Strippingzone, zum Zurückgewinnen von Wasser als ein Bodenprodukt und von einer dampfförmigen Fraktion, welche Methylacetat enthält.

6. Verfahren gemäß Anspruch 5, weiterhin **gekennzeichnet durch** ein Zurückführen der dampfförmigen Fraktion als einen Rückfluss in die Trennzone.

7. Verfahren für die Herstellung einer aromatischen Carboxylsäure durch eine Flüssigphasenoxidation eines Vorläufers jener Säure in der Essigsäure in Anwesenheit eines Katalysatorsystems, wobei dieses Verfahren umfasst:
(a) ein Überkopfentfernen aus einer Oxidationsreaktion eines dampfförmigen Stromes, der enthält; die Essigsäure, Wasser, das bei der Oxidationsreaktion erzeugt worden ist, und Methylacetat, das als ein Nebenprodukt bei der Oxidationsreaktion gebildet worden ist;
(b) ein Abzweigen aus dem Überkopfdampfstrom einer ersten flüssigen Phasenkomponente, welche die Essigsäure, Wasser und Methylacetat enthält;
(c) eine azeotrope Destillation dieser ersten flüssigen Phasenkomponente in Anwesenheit eines organischen Schleppmittels, das ausgewählt wird zwischen n-Propylacetat und Isobutylacetat, um eine zweite flüssige Phasenkomponente zu erzeugen, welche die Essigsäure umfasst, welche einen verminderten Wassergehalt relativ zu dem Wassergehalt in der ersten flüssigen Phase aufweist, sowie eine dampfförmige Phasenkomponente, welche das organische Schleppmittel, Wasser und Methylacetat enthält;
(d) ein Kondensieren der dampfförmigen Phasenkomponente unter Bedingungen, bei welchen das organische Schleppmittel und das Wasser kondensieren, während ein wesentlicher Teil des Methylacetats in der Dampfphase verbleibt;
(e) ein Wiedergewinnen des Methylacetats in der Dampfphase;
(f) eine Trennung des organischen Schleppmittels und des Wasserkondensats aus dem Schritt (d) in eine organische Phase und in eine wässrige Phase;
(g) ein Zurückführen der organischen Phase in die Säule; und
(h) ein Zurückführen von mindestens einem Teil des zurückgewonnenen Methylacetats zu der Oxidationsreaktion in der Flüssigphase.

8. Verfahren gemäß Anspruch 7, bei welchem die aromatische Carboxylsäure eine Terephthalsäure ist und der Vorläufer von dieser Säure Paraxylen ist.

## Revendications

1. Un procédé pour la distillation azéotropique d'une charge dans lequel la charge comprend de l'acide acétique, de l'eau et une impureté d'ester qui est de l'acétate de méthyle, qui comprend:
(a) la réalisation de la distillation azéotropique en présence d'un entraîneur organique sélectionné parmi l'acétate de n-propyle et l'acétate d'iso-butyle pour produire un composant en phase liquide comprenant ledit acide acétique possédant un contenu d'eau réduit par rapport au contenu d'eau dans la charge et un composant en phase vapeur qui contient l'entraîneur organique, de l'eau et de l'acétate de méthyle;
(b) la condensation du composant en phase vapeur;
(c) la séparation du condensé en (1) une phase organique comprenant principalement l'entraîneur avec des quantités mineures d'eau et d'acétate de méthyle et (2) une phase aqueuse comprenant principalement de l'eau avec des quantités mineures d'entraîneur et d'acétate de méthyle; et
(d) le renvoi de la phase organique vers la colonne de distillation, **caractérisé par** le contrôle de la condensation du composant en phase vapeur en amont de la séparation de la phase condensée de sorte que l'acétate de méthyle et une quantité mineure d'entraîneur restent dans la phase vapeur.

2. Le procédé de la revendication 1 **caractérisé en outre par** l'extraction simultanée de la phase aqueuse et de la phase vapeur dans une zone d'extraction pour récupérer de l'eau comme produit de fond, une fraction liquide qui est riche en entraîneur, et une fraction vapeur comprenant de l'acétate de méthyle.

3. Le procédé de la revendication 2 **caractérisé en outre par** la condensation de la fraction vapeur et le recyclage d'au moins une partie de ladite fraction vapeur condensée comme reflux vers la zone d'extraction.

4. Un procédé pour la distillation azéotropique d'une charge dans lequel la charge comprend de l'acide acétique, de l'eau et une impureté d'ester qui est de l'acétate de méthyle, qui comprend:
(a) la réalisation de la distillation azéotropique en présence d'un entraîneur organique sélectionné parmi l'acétate de n-propyle et l'acétate d'iso-butyle dans une zone de distillation pour produire un composant en phase liquide comprenant ledit acide acétique possédant un contenu d'eau réduit par rapport au contenu d'eau dans la charge et un composant en phase vapeur qui contient l'entraîneur organique, de l'eau et de l'acétate de méthyle;
(b) **caractérisé par** le fait de faire passer le composant en phase vapeur vers une zone de séparation incorporée et d'y séparer et de récupérer une fraction substantielle d'acétate de méthyle sous forme de vapeur tout en recueillant simultanément de l'eau, de l'entraîneur et une quantité mineure d'acétate de méthyle en phase liquide;
(c) la séparation de la phase liquide résultante en (1) une phase organique comprenant principalement l'entraîneur avec des quantités mineures d'eau et d'acétate de méthyle et (2) une phase aqueuse comprenant principalement de l'eau avec une quantité mineure d'acétate de méthyle; et
(d) le renvoi de la phase organique vers la zone de distillation.

5. Le procédé de la revendication 4 **caractérisé en outre par** l'extraction de la phase aqueuse dans une zone d'extraction pour récupérer de l'eau comme produit de fond et une fraction vapeur comprenant de l'acétate de méthyle.

6. Le procédé de la revendication 5 **caractérisé en outre par** le recyclage de la fraction vapeur comme reflux vers la zone de séparation.

7. Un procédé pour produire un acide carboxylique aromatique par oxydation en phase liquide d'un précurseur dudit acide dans de l'acide acétique en présence d'un système catalyseur, qui comprend le fait de:
(a) retirer de la réaction d'oxydation un courant de vapeur de tête qui comprend l'acide acétique, de l'eau produite dans la réaction d'oxydation et de l'acétate de méthyle formé comme sous-produit de la réaction d'oxydation;
(b) dériver du courant de vapeur de tête un premier composant en phase liquide qui contient l'acide acétique, de l'eau et de l'acétate de méthyle;
(c) distiller de manière azéotropique ledit premier composant en phase liquide en présence d'un entraîneur organique sélectionné parmi l'acétate de n-propyle et l'acétate d'iso-butyle pour produire un second composant en phase liquide comprenant ledit acide acétique possédant un contenu d'eau réduit par rapport au contenu d'eau dans la première phase liquide et un composant en phase vapeur qui contient l'entraîneur organique, de l'eau et de l'acétate de méthyle;
(d) condenser le composant en phase vapeur sous des conditions telles que l'entraîneur organique et l'eau se condensent alors qu'une portion substantielle de l'acétate de méthyle reste en phase vapeur;
(e) récupérer ledit acétate de méthyle dans la phase vapeur;
(f) séparer l'entraîneur organique et l'eau condensée de l'étape (d) en une phase organique et une phase aqueuse;
(g) renvoyer ladite phase organique vers la colonne; et
(h) renvoyer au moins une partie dudit acétate de méthyle récupéré vers la réaction d'oxydation en phase liquide.

8. Le procédé de la revendication 7 dans lequel l'acide carboxylique aromatique est de l'acide téréphtalique, le précurseur dudit acide est du paraxylène.
